# EUROPÄISCHE PATENTSCHRIFT

(11) **EP 0 636 883 B1**
(45) Veröffentlichungstag und Bekanntmachung des Hinweises auf die Patenterteilung: **08.05.2002**
(21) Anmeldenummer: 94110315.2
(22) Anmeldetag: 02.07.1994
(51) Int. Cl.: G01N 33/53, G01N 33/555, G01N 33/569, G01N 33/80, G01N 33/577

(54) **Verfahren zur Verstärkung der agglutinierenden Wirkung von Antikörpern und zur Verhinderung des Klebeeffekts bei diagnostischen Agglutinationstests unter Anwendung von Antikörpern**
Method for amplifying the agglutinating activity of antibodies and for inhibiting adhesive effects in diagnostic agglutination tests using antibodies
Méthode pour l'amplification de l'activité, agglutination d'anticorps et pour l'inhibition des effets adhésifs dans des essais d'agglutination diagnostiques utilisants des anticorps

(30) Priorität: 31.07.1993 DE 4325805
(43) Veröffentlichungstag der Anmeldung: 01.02.1995
(73) Patentinhaber: BIOTEST PHARMA GMBH, D-63303 Dreieich (DE)
(72) Erfinder: Ernst, Manfred, Dr., D-55257 Budenheim (DE); Möller, Wolfgang, Dr., D-61440 Oberursel (DE); Hies, Henry, D-63322 Rödermark (DE)
(74) Vertreter: Beil, Hans Christoph, Dr.

(56) Entgegenhaltungen:
- EP-A- 0 242 082
- EP-A- 0 260 903
- EP-A- 0 281 327
- EP-A- 0 525 916
- DATABASE WPI Section Ch, Week 7720, Derwent Publications Ltd., London, GB; Class B04, AN 77-35388 & JP-A-52 044 229 (FUJIZOKI PHARM KK.) 7. April 1977
- DATABASE WPI Section Ch, Week 8206, Derwent Publications Ltd., London, GB; Class B04, AN 82-11313 & SU-A-825 013 (V. N. MILYUTIN.) 30. April 1981
- JOURNAL OF IMMUNOLOGICAL METHODS, Bd.85, Nr.1, 1985, AMSTERDAM, NL. Seiten 409 - 419 J. G. KENNA ET AL. 'Methods for reducing non-specific antibody binding in enzyme-linked immunosorbent assays.'
- DATABASE WPI Section Ch, Week 9250, Derwent Publications Ltd., London, GB; Class B04, AN 92-412158 & JP-A-4 309 864 (HITACHI CHEM. CO. LTD.) 2. November 1992

## Beschreibung

Die vorliegende Erfindung betrifft ein Verfahren, bei diagnostischen Agglutinationstests die agglutinierende Wirkung von Antikörpern zu verstärken und/oder das Auftreten des Klebeeffekts zu verhindern, indem man dem Reaktionsmedium Casein zusetzt. Dadurch können Agglutinate deutlich besser abgelesen werden, da die letzte Verdünnungsstufe eindeutig erkannt wird. Das Verfahren eignet sich insbesondere sowohl für die passive als auch die aktive Hämagglutination.

Jeder Antikörper besitzt die Fähigkeit, sich an sein korrespondierendes Antigen zu binden. Die Erscheinungsform der Antigen-Antikörper-Reaktion ist dabei weitgehend von der Antigenstruktur abhängig.

Lösliche Antigene werden als Immunkomplexe präzipitiert, wenn sie eine gewisse Größe erreicht haben.

Bei der Agglutination, die vom Grundprinzip mit dem Rekationsverlauf der Präzipitation identisch ist, werden membranständige oder in anderer Weise fixierte Antigene spezifisch verklumpt.

Bei der direkten (aktiven) Agglutination tragen die zu agglutinierenden Partikel (z.B. Eryhtrozyten, Bakterien oder Latexpartikel) ihren Reaktionspartner, das Antigen, von vorneherein auf ihrer Oberfläche, während bei der passiven Hämagglutination normalerweise lösliche Antigene an z.B. Erythrozyten immobilisiert werden.

Ursache der Agglutination ist eine Vernetzung der Oberflächen-Antigen-Moleküle verschiedener Partikel durch Immunglobulin-Moleküle. Diese Vernetzung setzt das Vorliegen zwei- (IgG) oder mehrwertiger (IgA, IgM) Liganden voraus. Je besser die Antikörper mit den Antigenen auf den verschiedenen Partikeln reagieren können, umso besser wird die Vernetzung sein. Daher hängt die Stärke der Agglutination weniger von der Beschaffenheit der Antikörper, sondern vielmehr von den zu agglutinierenden Partikeln bzw. ihren Wechselwirkungen ab.

Die Agglutination läuft gegen Abstoßungskräfte ab, die z.B. durch gleichgerichtete Überschußladungen auf den Zellmembranen der Erythrozyten oder Bakterien bedingt sind.

Die Agglutination korpuskulärer Antigene spielt in vitro bei diagnostischen Verfahren eine große Rolle und wird unter anderem bei der Serodiagnostik zur Bestimmung von Infektionskrankheiten oder auch der Blutgruppen-Antikörper oder -Antigene eingesetzt.

So wird z.B. bei der passiven Hämagglutination das lösliche Antigen auf Erythrozyten als Träger fixiert. Die durch die zu bestimmenden Antikörper vermittelte Agglutination der Erythrozyten wird beobachtet. Auf diese Weise lassen sich Antikörperkonzentrationen quantitativ in Form von Titerstufen bestimmen.

Die passive Hämagglutination wird z.B. zur Bestimmung von Antikörpern gegen Escherichia Coli, Klebsiella pneumoniae, Staphylococcus faecalis, Streptococcus pyogenes, Streptococcus viridans und Pseudomonas aeruginosa in Immunglobulin-Präparationen aus Human- oder Rinder-Blut oder -Milch eingesetzt.

Das typische Testverfahren zur Bestimmung antibakterieller Antikörper verläuft dabei folgendermaßen:

Die Bakterien werden gezüchtet, abgetötet, und die löslichen Oberflächen-Antigene werden isoliert. Die Antigene werden mit Erythrozyten inkubiert und entweder kovalent oder durch Adsorption gebunden.
Die zu analysierende Antikörper-haltige Probe wird 30 min. bei 56°C inkubiert, um noch vorhandenes Komplement zu inaktivieren und anschließend mit Puffer, der gegebenenfalls Albumin enthält, verdünnt. Auf einer Mikrotiterplatte wird die Probe zu einer geometrischen Verdünnungsreihe verdünnt. Zu jeder verdünnten Probe werden antigenbeladene Erythrozyten zugegeben und 2 Stunden bei 37°C inkubiert. Danach werden die Platten in Schräglage gebracht und 3 mal im Abstand von 15 min. gedreht. Anschliessend wird die Agglutination beurteilt, wobei man eine negative Reaktion an dem zungenförmigen Auslaufen der Erythrozyten nach unten erkennt.

Mit zunehmender Verdünnung der Probe wird die Agglutination immer schwächer. Die Agglutination wird nach dem Punktesystem beurteilt:
- ++++: starke Agglutination
- +++: starke Agglutination, aber nur größere Erythrozyten-Zusammenballungen
- ++: grobkörnige Agglutination über den ganzen Brunnen verteilt
- +: feinkörnige Agglutination
- +/-: Übergangsphase zwischen feinkörniger Agglutination und negativer Reaktion
- -: keine Agglutination

Als Titer wird der reziproke Wert der letzten positiven (+/-) Verdünnungsstufe gewertet.

Die Beurteilung der letzten positiven Titerstufe ist sehr schwierig, da oft schon mehrere Titerstufen zuvor nur noch eine schwache Agglutination zu sehen ist, so daß eine Trennung positive/negative Reaktion nicht mehr eindeutig ist. Damit wird die Bestimmung der Titer sehr subjektiv und ist u.U. von der beurteilenden Person abhängig.

Ein weiteres Problem bei der diagnostischen Anwendung der Agglutination ist der sogenannte Klebeeffekt. Dabei werden die Agglutinate durch Interaktionen mit den Oberflächen der Gefäße, in denen die Agglutinationsreaktion abläuft, an den Gefäßwandungen festgehalten und können daher nicht richtig abgelesen werden.

Dieses Problem tritt besonders bei dem typischen Testablauf zur Bestimmung eines Blutgruppenantigens durch Agglutination der Erythrozyten (aktiver Agglutinationstest) auf, wie er im folgenden beschrieben wird:

Die zu untersuchenden Erythrozyten werden zu einer etwa 5 bis 10%igen Suspension in 0,9% NaCl-Lösung aufgeschwemmt. In entsprechend beschriftete Röhrchen wird je 1 Tropfen einer Anti-A-, Anti B- und Anti-A,B-Antikörper-Lösung gegeben. Anschließend wird in jedes Röhrchen 1 Tropfen der Erythrozytenaufschwemmung hinzugegeben und durch leichtes Aufschütteln gemischt. Die Röhrchen werden bei 1000 U/min zentrifugiert oder 20-30 Minuten bei Zimmertemperatur inkubiert. Auf dem Röhrchenboden bilden sich kleine Zellknöpfchen. Unter leichtem Aufschütteln wird daraus die Agglutinationsstärke abgelesen.

Die Agglutination wird nach einem Bewertungssystem beurteilt, bei dem die auf dem Röhrchenboden liegenden Zellknöpfchen in einer Skala von negativ bis vierfach positiv bezeichnet werden.
- ++++: der Zellknopf bleibt ein großes Agglutinat
- +++: der Zellknopf zerfällt in mehrere Agglutinate
- ++: der Zellknopf zerfällt in mehrere kleinere Agglutinate
- +: der Zellknopf zerfällt in kleine Granulate, die aber eindeutig noch als kleine Agglutinate zu erkennen sind
- +/-: zweifelhafte kleine Agglutinate
- -: keine Agglutination

Als Titer wird der reziproke Wert der letzten positiven (+/-) Verdünnungsstufe gewertet.

Die Beurteilung der Agglutinationsstärke wird bei manchen Blutgruppenreagenzien durch den Klebeeffekt erschwert, so daß auch hier eine eindeutige Zuordnung positive/negative Reaktion sehr schwierig ist.

Um die obengenannten Probleme zu lösen, wurde bisher zur Verstärkung der Agglutination von Erythrozyten dem Puffermedium Albumin, Dextran oder Gelatine bzw. Albumin und fötales Kälberserum, FCS, zur Verhinderung des Klebeeffekts zugesetzt. Mit diesen Reagenzien, welche die negativen Oberflächenladungen der Zellen neutralisieren, so daß die Erythrozyten besser zusammenkommen, können jedoch noch keine befriedigenden Ergebnisse, insbesondere bei der Bestimmung von bakteriellen Antigenen (passive Agglutination), erzielt werden.

Database WPI, Sec. CH, Week 7720, Derwent Publ., London GB, Class B04, AN 77-35388 beschreibt ein Verfahren zur Durchführung der Agglutinationsreaktion, wobei statt Schafserythrozyten Geflügelerythrozyten eingesetzt werden, was zu einer Zeitersparnis führen soll. Gegebenenfalls kann der Testlösung Gelatine, Gummi arabicum oder Casein in Mengen von 0,25 g/100 ml zugesetzt werden.

Database WPI, Sec. Ch, Week 8206, Derwent-Publ., London GB, Class B054, AN 82-11313 beschreibt ein Verfahren zur Vereinfachung der passiven Hämagglutination, wobei die Titerplatten aus Polystyrol vor der Reaktion in eine 1%ige Lösung aus Albumin oder Casein getaucht werden. Nach dem Waschen sollen unspezifische Bindungen auf diese Weise verhindert werden.

J. Immun. Meth., Bd. 85, S. 409-419 betrifft ein Verfahren zur Verhinderung unspezifischer Reaktionen bei ELISA-Tests, indem die Miktrotiterplatten u.a. mit Casein behandelt werden, um so unspezifische Bindungsstellen zu blockieren.

EP-A 0 281 327 sowie Database WPI, Sec. Ch, Week 9250, Derwent Publ., London GB, Class B04, AN 92-412158 betreffen ebenfalls Verfahren zur Verhinderung unspezifischer Reaktionen bei Agglutinationstests, wobei letztere durch Blockade der Oberflächen der Träger (z.B. Latexpartikel) blockiert werden.

Nirgends ist allerdings beschrieben, wie eine Verstärkung der Agglutination oder die Vermeidung eines Klebeeffekts erfolgen kann.

Aufgabe vorliegender Erfindung ist es daher, ein Verfahren zu entwickeln, durch welches die Agglutinationsreaktion bei der diagnostischen Anwendung von Antikörpern so verstärkt und/oder der auftretende Klebeeffekt verhindert werden kann, daß eine hohe Ablesegenauigkeit möglich ist, um zu besser reproduzierbaren, eindeutigen Ergebnissen zu gelangen.

Diese Aufgabe wird erfindungsgemäß dadurch gelöst, daß man dem Reaktionsmedium, insbesondere dem Antikörper-haltigen Medium, Casein zusetzt.

Überraschenderweise wurde gefunden, daß durch die Zugabe von Casein zur Probe zum einen eine deutlich stärkere Agglutination erfolgt und zum anderen der Klebeeffekt verhindert werden kann. Dies führt zu einer deutlich verbesserten Ablesbarkeit der Agglutinate, da die letzte Verdünnungsstufe eindeutig erkannt werden kann.

Die Verhinderung des Klebeeffekts wirkt sich darüberhinaus insbesondere bei Blutgruppentests zur Bestimmung von Erythrozytenantigenen bei der aktiven Hämagglutination aus, da das Blutgruppen(-Antikörper)-Reagenz, welches die teuerste Komponente bei diesen Tests ist, stärker verdünnbar ist, was neben der besseren Reproduzierbarkeit der Ergebnisse auch einen erheblichen wirtschaftlichen Vorteil darstellt.

Als erfindungsgemäß einzusetzendes Casein kann dabei die natürliche Mischung von alpha-, beta- und kappa-Casein, aber auch die einzelnen Komponenten alleine verwendet werden, welches vorzugsweise bovinen Ursprungs ist. Besonders bevorzugt wird ein Casein, welches aus Rindermilch in an sich bekannter Weise durch Säurefällung bei pH 4,5 gewonnen und bei neutralem pH-Wert wieder gelöst wird.

Das Casein wird dabei in Mengen von 5 bis 20 g/l und insbesondere 10 g/l, zur Grundverdünnung der Antikörperlösung zugesetzt. Dabei können die Nachweisreaktionen sowohl bei der passiven als auch bei der aktiven Hämagglutination mit geeigneten Antikörperlösungen durchgeführt werden. Als Antikörper sind jeweils dem Antigen entsprechende Immunglobuline, wie z.B. monoklonale, insbesondere humane oder murine, sowie polyklonale, insbesondere bovine oder humane Antikörper einsetzbar. Diese können vom IgA-, insbesondere vom IgM- oder vom IgG-Typ bzw. Mischungen hiervon sein. Das erfindungsgemäße Verfahren ist insbesondere geeignet zur Bestimmung bakterieller Antigene, wie z.B. Escherichia Coli, Klebsiella pneumoniae, Staphylococcus faecalis, Streptococcus pyogenes, Streptococcus viridans und Pseudomonas aeruginosa, bei der passiven Hämagglutination sowie zum Nachweis von Blutgruppen-Antigenen (z.B. RhD, A oder B) bei der aktiven Hämagglutination, wobei die entsprechenden obengenannten Antikörper und Casein in den angegebenen Mengen eingesetzt werden. Als Puffer werden dem Fachmann bekannte Medien verwendet.

Durch den erfindungsgemäßen Zusatz von Casein werden auch bei geringen Mengen wesentlich bessere Ergebnisse erzielt als z.B. durch das bisher verwendete Albumin oder FCS. Es handelt sich also bei der Wirkung von Casein nicht um die für Albumin bekannte einfache Beladung spezifischer Antigene mit Protein, sondern um einen Wirkmechanismus, der die Verstärkung der Agglutination beim Nachweis der verschiedensten Antigene allgemein bedingt. Dadurch muß der Zusatz nur der Grundverdünnung, und nicht - wie im Falle von Albumin - auch weiteren Verdünnungen hinzugefügt werden.

Mit dem erfindungsgemäßen Verfahren ist es daher möglich, auf einfache, kostengünstige und wirtschaftliche Weise nunmehr definitive, sicher reproduzierbare Ergebnisse bei der diagnostischen Agglutination unter Anwendung von geeigneten Antikörpern zu erzielen.

Die Erfindung wird anhand der nachfolgenden Beispiele näher erläutert.

### Beispiel 1

Eine IgG-, IgA- und IgM-haltige Fraktion aus menschlichem Plasma mit einem Gehalt von 36 g/l IgG, 6 g/l IgA und 6 g/l IgM wird mit Rinder-Casein versetzt und die antibakterielle Antikörperaktivität gegen E. Coli in der passiven Hämagglutination gemessen. Die Ergebnisse sind in Tabelle 1 angegeben.

**Tabelle 1**

| Verdünnungsstufe | Agglutination ohne Casein (Vergl.) | Agglutination mit 10g/l Casein (Erf.) | Agglutination mit 20g/l Casein (Erf.) |
|---|---|---|---|
| 1 : 20 | ++++ | ++++ | ++++ |
| 1 : 40 | +++ | ++++ | ++++ |
| 1 : 80 | ++ | ++++ | ++++ |
| 1 : 160 | ++ | ++++ | ++++ |
| 1 : 320 | + | +++ | ++++ |
| 1 : 640 | + | +++ | +++ |
| 1 : 1280 | + | +++ | ++ |
| 1 : 2560 | + | ++ | ++ |

Die Agglutination war deutlich stärker in den Proben, die mit Casein versetzt worden waren.
Die Höhe des Titers wird durch die Zugabe des Caseins nicht beeinflußt, ist aber aufgrund der stärkeren Agglutination deutlich besser ablesbar.

Als Casein wurde dabei (wie auch in den folgenden Beispielen) die natürliche Mischung von alpha-, beta- und kappa-Casein bovinen Ursprungs eingesetzt.

### Beispiel 2

Zu einer Probe mit 55 g/l polyklonalen IgG-Antikörpern und 5,3 g/l polyklonalen IgM-Antikörpern aus Rinderkolostralmilch werden steigende Mengen Albumin (Vergleich) bzw. Casein (Erfindung) zugegeben.

Die passive Hämagglutination zur Bestimmung der Antikörpertiter gegen E. coli und gegen Staphylococcus aureus wurde wie in Beispiel 1 durchgeführt. Die Ergebnisse sind in nachfolgender Tabelle 2 zusammengefaßt.

**Tabelle 2**

| Probe | Verdünnungsstufe | Agglutination E. Coli | Agglutination Staph. aureus |
|---|---|---|---|
| o. Zusatz | 1 : 20 | +++ | +++ |
| (Vergleich) | 1 : 40 | +++ | +++ |
| | 1 : 80 | ++ | ++ |
| | 1 : 160 | ++ | ++ |
| | 1 : 320 | ++ | + |
| | 1 : 640 | + | +/- |
| | 1 : 1280 | - | - |
| | Summe: | 13 | 11 |
| 10 g/l Albumin | 1 : 20 | +++ | +++ |
| (Vergleich) | 1 : 40 | +++ | +++ |
| | 1 : 80 | ++ | ++ |
| | 1 : 160 | ++ | ++ |
| | 1 : 320 | ++ | + |
| | 1 : 640 | + | +/- |
| | 1 : 1280 | - | - |
| | Summe: | 13 | 11 |
| 30 g/l Albumin | 1 : 20 | ++++ | ++++ |
| (Vergleich) | 1 : 40 | +++ | ++++ |
| | 1 : 80 | ++ | +++ |
| | 1 : 160 | ++ | ++ |
| | 1 : 320 | ++ | +/- |
| | 1 : 640 | + | - |
| | 1 : 1280 | - | - |
| | Summe: | 14 | 13 |
| 10 g/l Casein | 1 : 20 | ++++ | ++++ |
| (Erfindung) | 1 : 40 | ++++ | ++++ |
| | 1 : 80 | ++++ | ++++ |
| | 1 : 160 | +++ | ++++ |
| | 1 : 320 | +++ | ++ |
| | 1 : 640 | ++ | +/- |
| | 1 : 1280 | - | - |
| | Summe: | 20 | 18 |

Wie hieraus ersichtlich ist, läßt sich auch durch Zugabe noch größerer Mengen Albumin die Agglutination nicht wesentlich weiter verstärken. Der Effekt von nur 10 g/l Casein wird bei weitem nicht erreicht.

### Beispiel 3

Eine bovines IgG, IgA und IgM enthaltende Präparation mit einem Gehalt von 50 g/l IgG, 5 g/l IgA und 6 g/l IgM wird mit 10 g/l Rinder-Casein versetzt und die antibakterielle Antikörperaktivität gegen Escherichia Coli, Klebsiella pneumoniae, Staphylococcus faecalis, Staphylococcus aureus, Streptococcus pyogenes, Streptococcus viridans und Pseudomonas aeruginosa in der passiven Hämagglutination gemessen. Die Ergebnisse sind in Tabelle 3 angegen.

**Tabelle 3**

| | Verdünnung | | | | | |
|---|---|---|---|---|---|---|
| Probe | 1:40 | 1:80 | 1:160 | 1:320 | 1:640 | 1:1280 |
| E. Coli | | | | | | |
| o. Casein(Vgl.) | ++++ | +++ | ++ | ++ | + | - |
| m. Casein(Erf.) | ++++ | ++++ | ++++ | +++ | ++ | - |
| | | | | | | |

| Ps. aeruginosa | | | | | | |
|---|---|---|---|---|---|---|
| o. Casein(Vgl.) | ++++ | ++++ | +++ | + | - | |
| m. Casein(Erf.) | ++++ | ++++ | +++ | +++ | - | |
| | | | | | | |

| Klebs. pneumoniae | | | | | | |
|---|---|---|---|---|---|---|
| o. Casein(Vgl.) | +++ | +++ | ++ | ++ | - | |
| m. Casein(Erf.) | +++ | +++ | +++ | +++ | ++ | - |
| | | | | | | |

| Staph. aureus | | | | | | |
|---|---|---|---|---|---|---|
| o. Casein(Vgl.) | +++ | +++ | ++ | - | | |
| m. Casein(Erf.) | ++++ | ++++ | +++ | - | | |
| | | | | | | |

| Strept. epidermidis | | | | | | |
|---|---|---|---|---|---|---|
| o. Casein(Vgl.) | +++ | +++ | ++ | ++ | + | - |
| m. Casein(Erf.) | ++++ | ++++ | ++++ | ++ | + | - |

Man kann deutlich erkennen, daß die Verstärkung der Agglutination mit Casein bei einer Vielzahl von insbesondere für den Menschen pathogenen Keimen erfolgt.

### Beispiel 4

Ein humaner monoklonaler IgM-Antikörper, BS 226, spezifisch für das Blutgruppenmerkmal RhD, wird mit RhD-positiven Erythrozyten in der direkten Agglutination getestet. Dabei werden die Zusätze fötales Kälberserum (FCS), Rinderserumalbumin (BSA) als Vergleich und Rinder-Casein gemäß vorliegender Erfindung in verschiedenen Konzentrationen eingesetzt, um den Klebeeffekt, wie er im unbehandelten Reagenz auftritt, zu verhindern. Tabelle 4 zeigt die Ergebnisse.

**Tabelle 4**

| | Verdünnung | | | | | |
|---|---|---|---|---|---|---|
| BS 226 | 1:2 | 1:4 | 1:8 | 1:16 | 1:32 | 1:64 |
| 10% FCS (Vgl.) | ++++ K | ++++ K | ++++ K | ++++ K | ++++ K | ++++(K) |
| 20% FCS (Vgl.) | ++++ K | ++++ K | ++++ K | ++++(K) | ++++(K) | ++++ |
| 30% FCS (Vgl.) | ++++ K | ++++(K) | ++++ | ++++ | ++++ | ++++ |
| 40% FCS (Vgl.) | ++++ | ++++ | ++++ | ++++ | ++++ | ++++ |
| 15% BSA (Vgl.) | ++++ K | ++++ K | ++++ K | ++++ K | ++++(K) | ++++(K) |
| 30% BSA (Vgl.) | ++++ K | ++++ K | ++++ K | ++++ K | ++++ K | ++++(K) |
| | | | | | | |
| 10g/l Casein (Erf.) | ++++ | ++++ | ++++ | ++++ | ++++ | ++++ |
| K = Klebeeffekt, (K) = schwacher Klebeeffekt | | | | | | |

Wie hieraus ersichtlich ist, kann durch Zusatz von 10 g/l Casein der Klebeeffekt deutlich besser verhindert werden, wie es vergleichbar nur bei dem bekannten Zusatz von mehr als 30% FCS möglich ist. Dabei ist der Zusatz von Casein deutlich preiswerter.

### Beispiel 5

Ein humaner monoklonaler IgM-Antikörper, BS 226, spezifisch für das Blutgruppenmerkmal RhD, wird mit RhD-positiven Erythrozyten in der direkten Agglutination getestet. Dabei wird der Zusatz von Casein in verschiedenen Konzentrationen auf die Wirksamkeit zur Verhinderung des Klebeeffekts überprüft (Tabelle 5).

**Tabelle 5**

| | Verdünnung | | | | | |
|---|---|---|---|---|---|---|
| BS 226 | 1:2 | 1:4 | 1:8 | 1:16 | 1:32 | 1:64 |
| 1 g/l Casein | ++++ K | ++++ K | ++++ K | ++++ K | ++++(K) | ++++(K) |
| 5 g/l Casein | ++++ K | ++++ K | ++++ K | ++++ K | ++++(K) | ++++ |
| 10 g/l Casein | ++++ | ++++ | ++++ | ++++ | ++++ | ++++ |
| K = Klebeeffekt, (K) = schwacher Klebeeffekt | | | | | | |

Durch Zusatz von 10 g/l Casein kann der Klebeeffekt wirksam verhindert werden.

### Beispiel 6

Ein humaner monoklonaler IgM-Antikörper, BS 226, spezifisch für das Blutgruppenmerkmal RhD, wird mit RhD-positiven Erythrozyten im direkten Agglutinationstest austitriert.
Dabei werden 10 g/l Casein zugesetzt, um den Klebeeffekt zu verhindern und die Verstärkung der Agglutination nachzuweisen (Tabelle 6).

**Tabelle 6**

| | Verdünnung | | | | | |
|---|---|---|---|---|---|---|
| BS 226 | 1:8 | 1:16 | 1:32 | 1:64 | 1:128 | 1:256 |
| ohne C*(Vgl.) | ++++ K | ++++ K | ++++ K | ++++ K | ++++ K | ++++ K |
| 10 g/l C*(Erf.) | ++++ | ++++ | ++++ | ++++ | ++++ | ++++ |

| | Verdünnung | | | | | |
|---|---|---|---|---|---|---|
| BS 226 | 1:512 | 1:1024 | 1:2048 | 1:4096 | 1:8000 | 1:16000 |
| ohne C* (Vgl.) | +++ K | +++ K | +++ K | ++ K | - | - |
| 10 g/l C*(Erf.) | ++++ | +++ | +++ | +++ | +++ | + |
| K = Klebeeffekt, (K) = schwacher Klebeeffekt | | | | | | |

| | | | | | | |
|---|---|---|---|---|---|---|
| * C = Casein | | | | | | |

Wie sich hieraus ergibt, kann durch 10 g/l Casein der Klebeeffekt verhindert werden. Zusätzlich wird eine Verstärkung der Agglutination und eine Verstärkung des Titers erreicht, wenn man die Probe austitriert.

### Beispiel 7

Ein humaner monoklonaler IgM-Antikörper, BS 232, spezifisch für das Blutgruppenmerkmal RhD, wird mit RhD-positiven Erythrozyten im direkten Agglutinationstest austitriert. Dabei werden 10 g/l Casein zugesetzt, um den Klebeeffekt zu verhindern und die Verstärkung der Agglutination nachzuweisen (Tab. 7)

**Tabelle 7**

| | Verdünnung | | | | | |
|---|---|---|---|---|---|---|
| BS 232 | 1:4 | 1:8 | 1:16 | 1:32 | 1:64 | 1:128 |
| ohne C*(Vgl.) | ++++ K | ++++ K | +++ K | + K | + K | - |
| 10 g/l C*(Erf.) | ++++ | ++++ | ++++ | ++++ | ++++ | ++++ |

| | Verdünnung | | | | | |
|---|---|---|---|---|---|---|
| BS 232 | 1:256 | 1:512 | 1:1024 | 1:2048 | 1:4096 | |
| ohne C* (Vgl.) | - | - | - | - | - | |
| 10 g/l C*(Erf.) | ++++ | +++ | + | + | + | |
| K = Klebeeffekt, (K) = schwacher Klebeeffekt | | | | | | |

| | | | | | | |
|---|---|---|---|---|---|---|
| * C = Casein | | | | | | |

Durch 10 g/l Casein kann der Klebeeffekt verhindert werden. Zusätzlich wird eine Verstärkung der Agglutination und eine Verstärkung des Titers erreicht, wenn man die Probe austitriert.

### Beispiel 8

Ein muriner monoklonaler IgM-Antikörper, BS 63, spezifisch für das Blutgruppenmerkmal A, wird mit A-positiven Erythrozyten im direkten Agglutinationstest austitriert.

Dabei werden 10 g/l Casein zugesetzt, um den Klebeeffekt zu verhindern und die Verstärkung der Agglutination nachzuweisen (Tab. 8).

**Tabelle 8**

| | Verdünnung | | | | | |
|---|---|---|---|---|---|---|
| BS 63 | 1:4 | 1:8 | 1:16 | 1:32 | 1:64 | 1:128 |
| ohne C* (Vgl.) | ++++ K | ++++ K | +++ K | + K | + | K - |
| 10g/l C*(Erf.) | ++++ | ++++ | ++++ | ++++ | ++++ | ++++ |

| | Verdünnung | | | | | |
|---|---|---|---|---|---|---|
| BS 63 | 1:256 | 1:512 | 1:1024 | 1:2048 | | 1:4096 |
| ohne C*(Vgl.) | - | - | - | - | | - |
| 10 g/l C*(Erf.) | ++++ | +++ | + | + | | + |
| K = Klebeeffekt, (K) = schwacher Klebeeffekt | | | | | | |

| | | | | | | |
|---|---|---|---|---|---|---|
| * C = Casein | | | | | | |

Durch 10 g/l Casein kann der Klebeeffekt verhindert werden. Zusätzlich wird eine Verstärkung der Agglutination und eine Verstärkung des Titers erreicht, wenn man die Probe austitriert.

## Patentansprüche

1. Verfahren zur Verstärkung der Agglutinationsreaktion und/oder zur Vermeidung des Klebeeffektes bei Agglutinationsreaktionen bei der diagnostischen Anwendung von Antikörpern, **dadurch gekennzeichnet, daß** man die Reaktion in Gegenwart einer Caseinlösung durchführt, wobei das Casein in einer Konzentration von 5 bis 20 g/l der Antikörperlösung vorliegt.

2. Verfahren nach Anspruch 1, **dadurch gekennzeichnet, daß** als Nachweisreaktion eine passive oder aktive Hämagglutination erfolgt.

3. Verfahren nach Anspruch 2, **dadurch gekennzeichnet, daß** bakterielle Antigene bei der passiven Hämagglutination bestimmt werden.

4. Verfahren nach Anspruch 2, **dadurch gekennzeichnet, daß** Blutgruppen-Antigene bei der aktiven Hämagglutination bestimmt werden.

5. Verfahren nach einem der Ansprüche 1 bis 4, **dadurch gekennzeichnet, daß** als Antikörper monoklonale Antikörper verwendet werden.

6. Verfahren nach Anspruch 5, **dadurch gekennzeichnet, daß** die Antikörper humane monoklonale Antikörper sind.

7. Verfahren nach Anspruch 5, **dadurch gekennzeichnet, daß** die Antikörper murine monoklonale Antikörper sind.

8. Verfahren nach einem der Ansprüche 1 bis 4, **dadurch gekennzeichnet, daß** als Antikörper polyklonale Antikörper verwendet werden.

9. Verfahren nach Anspruch 8, **dadurch gekennzeichnet, daß** die polyklonalen Antikörper vom Menschen stammen.

10. Verfahren nach Anspruch 8, **dadurch gekennzeichnet, daß** die polyklonalen Antikörper vom Rind stammen.

11. Verfahren nach einem der Ansprüche 1 bis 10, **dadurch gekennzeichnet, daß** man Antikörper vom IgM-Typ verwendet.

12. Verfahren nach einem der Ansprüche 1 bis 10, **dadurch gekennzeichnet, daß** man Antikörper vom IgG-Typ verwendet.

13. Verfahren nach einem der Ansprüche 1 bis 12, **dadurch gekennzeichnet, daß** man bovines Casein verwendet.

14. Verfahren nach Anspruch 13, **dadurch gekennzeichnet, daß** man ein durch Säurefällung aus Rindermilch gewonnenes Casein verwendet.

## Claims

1. Method of amplifying the agglutination reaction and/or avoiding the adhesive effect in agglutination reactions using antibodies for diagnostic purposes, **characterized in that** the reaction is carried out in the presence of a casein solution in which the casein is in a concentration of 5 to 20 g/l of antibody solution.

2. Method according to Claim 1, **characterized in that** a passive or active haemagglutination occurs as the detection reaction.

3. Method according to Claim 2, **characterized in that** bacterial antigens are determined in the passive haemagglutination.

4. Method according to Claim 2, **characterized in that** blood group antigens are determined in the active haemagglutination.

5. Method according to one of Claims 1 to 4, **characterized in that** the antibodies used are monoclonal antibodies.

6. Method according to Claim 5, **characterized in that** the antibodies are human monoclonal antibodies.

7. Method according to Claim 5, **characterized in that** the antibodies are murine monoclonal antibodies.

8. Method according to one of Claims 1 to 4, **characterized in that** the antibodies used are polyclonal antibodies.

9. Method according to Claim 8, **characterized in that** the polyclonal antibodies are of human origin.

10. Method according to Claim 8, **characterized in that** the polyclonal antibodies are of bovine origin.

11. Method according to one of Claims 1 to 10, **characterized in that** antibodies of the IgM type are used.

12. Method according to one of Claims 1 to 10, **characterized in that** antibodies of the IgG type are used.

13. Method according to one of Claims 1 to 12, **characterized in that** bovine casein is used.

14. Method according to Claim 13, **characterized in that** the casein used is obtained from cow's milk by acid precipitation.

## Revendications

1. Procédé pour renforcer la réaction d'agglutination et/ou pour éviter l'effet d'adhésion dans les réactions d'agglutination dans l'utilisation diagnostique d'anticorps, **caractérisé en ce que** l'on conduit la réaction en présence d'une solution de caséine, la caséine étant présente en une concentration de 5 à 20 g/l de la solution d'anticorps.

2. Procédé selon la revendication 1, **caractérisé en ce que** la réaction de mise en évidence est une hémagglutination passive ou active.

3. Procédé selon la revendication 2, **caractérisé en ce que** des antigènes bactériens sont déterminés dans l'hémagglutination passive.

4. Procédé selon la revendication 2, **caractérisé en ce que** des antigènes de groupes sanguins sont déterminés dans l'hémagglutination active.

5. Procédé selon l'une des revendications 1 à 4, **caractérisé en ce que** des anticorps monoclonaux sont utilisés comme anticorps.

6. Procédé selon la revendication 5, **caractérisé en ce que** les anticorps sont des anticorps monoclonaux humains.

7. Procédé selon la revendication 5, **caractérisé en ce que** les anticorps sont des anticorps monoclonaux murins.

8. Procédé selon l'une des revendications 1 à 4, **caractérisé en ce que** des anticorps polyclonaux sont utilisés comme anticorps.

9. Procédé selon la revendication 8, **caractérisé en ce que** les anticorps polyclonaux sont issus de l'homme.

10. Procédé selon la revendication 8, **caractérisé en ce que** les anticorps polyclonaux sont issus du boeuf.

11. Procédé selon l'une des revendications 1 à 10, **caractérisé en ce que** l'on utilise des anticorps du type IgM.

12. Procédé selon l'une des revendications 1 à 10, **caractérisé en ce que** l'on utilise des anticorps du type IgG.

13. Procédé selon l'une des revendications 1 à 12, **caractérisé en ce que** l'on utilise la caséine bovine.

14. Procédé selon la revendication 13, **caractérisé en ce que** l'on utilise une caséine obtenue à partir du lait de vache par précipitation à l'acide.
